# EUROPEAN PATENT SPECIFICATION

(11) **EP 1 626 727 B1**
(45) Date of publication and mention of the grant of the patent: **31.12.2008**
(21) Application number: 04709051.9
(22) Date of filing: 06.02.2004
(51) Int. Cl.: A61K 31/5415, A61P 25/00

(54) **USE OF 2-CYANO-10- (2-METHYL-3- (METHYLAMINO)- PROPYL) PHENOTHIAZINE OR A PHARMACEUTICALLY ACCEPTABLE SALT THEREOF AS A MEDICAMENT**
VERWENDUNG VON 2-CYANO-10- (2-METHYL-3- (METHYLAMINO)- PROPYL)-PHENOTHIAZIN ODER EINEM PHARMAZEUTISCH AKZEPTABLEN SALZ DAVON ALS ARZNEIMITTEL
UTILISATION, EN TANT QUE MEDICAMENT DE 2-CYANO-10-(2-METHYL-3-(METHYLAMINO)-PROPYL) PHENOTHIAZINE OU UN SEL DE CELUI-CI PHARMACEUTIQUEMENT ACCEPTABLE

(30) Priority: 07.02.2003 FR 0301440; 02.04.2003 US 459812 P
(43) Date of publication of application: 22.02.2006
(73) Proprietor: Aventis Pharmaceuticals Inc., Bridgewater, New Jersey 08807 (US)
(72) Inventor: DIB, Michel, F-75013 Paris (FR); HAMEG, Ahcene, F - 95240 CORMEILLES EN PARISIS (FR)
(74) Representative: Rousseau, Pierrick Edouard
(86) International application number: PCT/US2004/003480
(87) International publication number: WO 2004/071512

(56) References cited:
- WO-A-01/12265
- WO-A-01/41769
- US-A- 2 877 224
- NAASSILA M ET AL: "CYMEMAZINE DECREASES ETHANOL INTAKE IN RATS AND CONVULSIONS DURING ETHANOL WITHDRAWAL SYNDROME IN MICE" PSYCHOPHARMACOLOGY, SPRINGER VERLAG, BERLIN, DE, vol. 140, no. 4, December 1998 (1998-12), pages 421-428, XP000908904 ISSN: 0033-3158
- HAMEG AHCÈNE ET AL: "Affinity of cyamemazine, an anxiolytic antipsychotic drug, for human recombinant dopamine vs. serotonin receptor subtypes." BIOCHEMICAL PHARMACOLOGY. ENGLAND 1 FEB 2003, vol. 65, no. 3, 1 February 2003 (2003-02-01), pages 435-440, XP002253341 ISSN: 0006-2952

## Description

Use of 2-cyano-10-(2-methyl-3-(methylamino)-propyl)phenothiazine to produce a medicament for the treatment of sleep disorders.

The present invention relates to the use of 2-cyano-10-(2-methyl-3-(methylamino)propyl)phenothiazine (I) or a pharmaceutically acceptable salt of this compound to produce a medicament for the treatment of sleep disorders, anxiety disorders (generalized anxiety, panic disorder, with or without agoraphobia, post-traumatic stress condition, anxiety disorder due to a general condition, adaptation disorder with an anxious mood, acute stress condition, nonspecific anxiety disorder, minor anxiety, substance-induced anxiety disorder, and the like), mood disorders (major depressive episode, manic episode, mixed episode, bipolar disorders, nonspecific mood disorder, nonspecific depressive disorder), mixed anxiety-depression disorder, acute and chronic psychotic states (schizophrenia, schizophreniform disorder, schizoaffective disorder, delusional disorder, brief psychotic disorder, substance-induced psychotic disorder, nonspecific psychotic disorder, psychotic disorder due to a general medical condition), behavioural disorders (agitation, aggressiveness, and the like), addiction to and withdrawal from a substance (nicotine, alcohol, benzodiazepine, cocaine, cannabis, hallucinogens, amphetamines), extrapyramidal events induced by antipsychotics (preventive and/or curative treatment), or symptomatic dimensions during acute or chronic psychotic states as monotherapy or in combination with other antipsychotics.

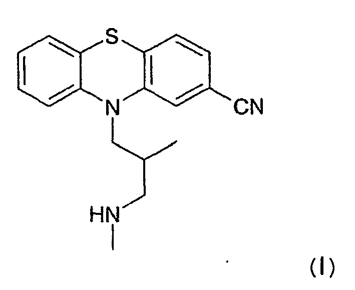

It is known from the prior art of 1959, GB 805 886, that products derived from 10-phenothiazine can be used as vegetative nervous system inhibitor. The process for producing 2-cyano-10-(2-methyl-3-(methylamino)propyl)phenothiazine (I) is disclosed in GB 805 886.

More particularly, the present invention relates to the use of 2-cyano-10-(2-methyl-3-(methylamino)propyl)phenothiazine (I) or a pharmaceutically acceptable salt of this compound to produce a medicament intended for the treatment of sleep disorders.

Sleep disorders affect approximately 30 to 35% of the population, according to an enquiry by G.D. Mellinger (Arch. Gen. Psychiatry, 1985, 42, 225-232).

This illness is currently treated mainly with hypnotic benzodiazepines or related benzodiazepines, H1 antihistamines or sedative neuroleptics. There are molecules in development which act on receptors of histamine H3 type or serotoninergic receptors of 5-HT2a type.

It has been found that 2-cyano-10-(2-methyl-3-(methylamino)propyl)phenothiazine (I) exhibits an advantageous binding profile with regard to the receptors with a very good affinity ratio with regard to 5-HT2a/D2 and an excellent selectivity with regard to the muscarinic M1 receptor in comparison with the other muscarinic M2 and M3 receptors. These results make it possible to affirm that 2-cyano-10-(2-methyl-3-(methylamino)propyl)phenothiazine has a very good tolerance profile with in particular fewer extrapyramidal effects and fewer anticholinergic effects. This is because, according to Can. J. Psychiatry, 2002, 47(1), 27-38, the risk of appearance of extrapyramidal events during antipsychotic treatment is inversely proportional to the degree of binding to the 5-HT2a receptors and to the 5-HT2a/D2 affinity ratio. Furthermore, 2-cyano-10-(2-methyl-3-(methylamino)propyl)phenothiazine exhibits a high affinity for 5-HT2c receptors, the role of which in anxiety disorders is currently well established.

The results of this study of binding to these various membrane receptors of central neuromediators of human origin are represented in Table 1.

**Table 1**

| Receptors | 2-Cyano-10-(2-methyl-3-(methylamino)-propyl)phenothiazine | |
|---|---|---|
| | IC₅₀ (nM) | Ki (nM) |
| D2 (h) | 31 | 12 |
| M₁ (h) | 21 | 17 |
| M₂ (h) | 368 | 251 |
| M₃ (h) | 5 490 | 3 920 |
| 5-HT_{1A} (h) | 460 | 184 |
| 5-HT_{2A} (h) | 9.0 | 1.5 |
| 5-HT_{2C} (h) | 23 | 8.5 |
| H₁ (h) | 22 | 9.3 |

These excellent results make it possible to say that the side effects will be reduced in comparison with the currently existing products.

The sedative activity of the product was determined with mice according to an actimetry test. The actimeter is a device composed of 6 transparent cages in which the animals are individually placed. Photoelectric cells make it possible to detect movements in the cages (by cutting the beam). The spontaneous motor activity is recorded for 10 minutes. The results are expressed in the mean form and in the form of percentage of activity with respect to the control batch. The results are expressed in Table 2.

**Table 2**

| | | | | |
|---|---|---|---|---|
| Doses mg/kg | 0.75 | 1 | 2 | 4 |
| % of activity/control | 61% | 63% | 26% | 9% |

Mention may in particular be made, as pharmaceutically acceptable salts, of the addition salts with inorganic acids, such as hydrochloride, sulphate, nitrate or phosphate, or organic acids, such as acetate, propionate, succinate, oxalate, benzoate, fumarate, maleate, methanesulphonate, isethionate, theophyllineacetate, salicylate, phenolphthalinate, methylenebis(β-hydroxynaphthoate) or derivatives from substitution of these derivatives.

The medicaments are composed of 2-cyano-10-(2-methyl-3-(methylamino)propyl)phenothiazine (I) or a pharmaceutically acceptable salt of this compound with a pharmaceutically acceptable acid, in the pure state or in the form of a composition in which it is combined with any other pharmaceutically compatible product, which can be inert or physiologically active. The medicaments according to the invention can be employed orally or parenterally.

The present invention relates to the use of 2-cyano-10-(2-methyl-3-(methylamino)propyl)phenothiazine (I) or a pharmaceutically acceptable salt of this compound for the preparation of pharmaceutical compositions.

Tablets, pills, powders (cachets, gelatin capsules) or granules can be used as solid compositions for oral administration. In these compositions, the active principle according to the invention is mixed with one or more inert diluents, such as starch, cellulose, sucrose, lactose or silica, under a stream of argon. These compositions can also comprise substances other than the diluents, for example one or more lubricating agents, such as magnesium stearate or talc, a coloring agent, a coating (dragées) or a glaze.

Pharmaceutically acceptable solutions, suspensions, emulsions, and syrups and elixirs comprising inert diluents, such as water, ethanol, glycerol, vegetable oils or liquid paraffin, can be used as liquid compositions for oral administration. These compositions can comprise substances other than the diluents, for example wetting, sweetening, thickening, flavouring or stabilizing products.

The sterile compositions for parenteral administration can preferably be suspensions, emulsions or aqueous or nonaqueous solutions. Water, propylene glycol, a polyethylene glycol, vegetable oils, in particular olive oil, injectable organic esters, for example ethyl oleate, or other suitable organic solvents can be employed as solvent or vehicle. These compositions can also comprise adjuvants, in particular wetting, isotonizing, emulsifying, dispersing and stabilizing agents. Sterilization can be carried out in several ways, for example by aseptic filtration, by incorporating sterilizing agents in the composition, by irradiation or by heating. They can also be prepared in the form of sterile solid compositions which can be dissolved at the time of use in sterile water or any other injectable sterile medium.

The doses depend on the desired effect, on the duration of the treatment and on the administration route used; they are generally between 10 and 300 mg per day orally for an adult with unit doses ranging from 10 to 300 mg of active substance.

Generally, the doctor will determine the appropriate dosage depending on the age, weight and all the other factors specific to the subject to be treated.

The following examples illustrate medicaments according to the invention:

### Example A

Tablets comprising a dose of 25 mg of active product are prepared according to the usual technique. These tablets have the following composition:

| | |
|---|---|
| Product | 25 mg |
| Lactose | 60 mg |
| Wheat starch | 45 mg |
| Hydrated silica | 4.5 mg |
| Alginic acid | 2.25 mg |
| Talc | 0.75 mg |
| Magnesium stearate | 0.90 mg |

### Example B

An injectable solution comprising 1 g of active product is prepared. This solution has the following composition:

| | |
|---|---|
| Product | 1 g |
| Ascorbic acid | 0.1 g |
| Monothioglycerol | 0.3 g |
| Polyethylene glycol 400 | 0.02 g |
| Water for Injections | q.s. for 100 ml |

The invention also relates to the process for the preparation of medicaments of use in the treatment of sleep disorders which consists in mixing 2-cyano-10-(2-methyl-3-(methylamino)propyl)phenothiazine (I) or its pharmaceutically acceptable salts with one or more compatible and pharmaceutically acceptable diluents and/or adjuvants.

## Claims

1. Use of 2-cyano-10-(2-methyl-3-(methylamino)-propyl)phenothiazine (I) and its pharmaceutically acceptable salts for preparing a medicament for the treatment of sleep disorders.

2. Use of 2-cyano-10-(2-methyl-3-(methylamino)-propyl)phenothiazine (I) and its pharmaceutically acceptable salts for preparing a medicament for the treatment of anxiety disorders.

3. Use of 2-cyano-10-(2-methyl-3-(methylamino)-propyl)phenothiazine (I) and its pharmaceutically acceptable salts for preparing a medicament for the treatment of mood disorders.

4. Use of 2-cyano-10-(2-methyl-3-(methylamino)-propyl)phenothiazine (I) and its pharmaceutically acceptable salts for preparing a medicament for the treatment of mixed anxiety-depression disorders.

5. Use of 2-cyano-10-(2-methyl-3-(methylamino)-propyl)phenothiazine (I) and its pharmaceutically acceptable salts for preparing a medicament for the treatment of an acute and chronic psychotic state.

6. Use of 2-cyano-10-(2-methyl-3-(methylamino)-propyl)phenothiazine (I) and its pharmaceutically acceptable salts for preparing a medicament for the treatment of addiction to and withdrawal from a substance.

7. Use of 2-cyano-10-(2-methyl-3-(methylamino)-propyl)phenothiazine (I) and its pharmaceutically acceptable salts for preparing a medicament for the treatment of extrapyramidal events induced by antipsychotics.

8. Use of 2-cyano-10-(2-methyl-3-(methylamino)-propyl)phenothiazine (I) and its pharmaceutically acceptable salts for preparing a medicament for the treatment of symptomatic dimensions during acute or chronic psychotic states as monotherapy or in combination with other antipsychotics.

9. Use according to any of Claims 1 to 8 wherein the medicament comprises 10 to 300 mg of 2-cyano-10-(2-methyl-3-(methylamino)propyl)phenothiazine (I) or its pharmaceutically acceptable salts.

10. Process for the preparation of a medicament of use for the treatment of sleep disorders, **characterized in that** 2-cyano-10-(2-methyl-3-(methylamino)propyl)phenothiazine or the pharmaceutically acceptable salts of this compound are mixed with one or more compatible and pharmaceutically acceptable diluents and/or adjuvants.

## Patentansprüche

1. Verwendung von 2-Cyano-10-(2-methyl-3-(methylamino)propyl)phenothiazin (I) und pharmazeutisch annehmbaren Salzen davon zur Herstellung eines Arzneimittels zur Behandlung von Schlafstörungen.

2. Verwendung von 2-Cyano-10-(2-methyl-3-(methylamino)propyl)phenothiazin (I) und pharmazeutisch annehmbaren Salzen davon zur Herstellung eines Arzneimittels zur Behandlung von Angststörungen.

3. Verwendung von 2-Cyano-10-(2-methyl-3-(methylamino)propyl)phenothiazin (I) und pharmazeutisch annehmbaren Salzen davon zur Herstellung eines Arzneimittels zur Behandlung von Gemütsstörungen.

4. Verwendung von 2-Cyano-10-(2-methyl-3-(methylamino)propyl)phenothiazin (I) und pharmazeutisch annehmbaren Salzen davon zur Herstellung eines Arzneimittels zur Behandlung von gemischten Angst-Depressions-Störungen.

5. Verwendung von 2-Cyano-10-(2-methyl-3-(methylamino)propyl)phenothiazin (I) und pharmazeutisch annehmbaren Salzen davon zur Herstellung eines Arzneimittels zur Behandlung eines akuten und chronischen psychotischen Zustands.

6. Verwendung von 2-Cyano-10-(2-methyl-3-(methylamino)propyl)phenothiazin (I) und pharmazeutisch annehmbaren Salzen davon zur Herstellung eines Arzneimittels zur Behandlung der Abhängigkeit von einer Substanz und des Entzugs einer Substanz.

7. Verwendung von 2-Cyano-10-(2-methyl-3-(methylamino)propyl)phenothiazin (I) und pharmazeutisch annehmbaren Salzen davon zur Herstellung eines Arzneimittels zur Behandlung von durch Antipsychotika induzierten extrapyramidalen Ereignissen.

8. Verwendung von 2-Cyano-10-(2-methyl-3-(methylamino)propyl)phenothiazin (I) und pharmazeutisch annehmbaren Salzen davon zur Herstellung eines Arzneimittels zur Behandlung von symptomatischen Dimensionen während akuter oder chronischer psychotischer Zustände als Monotherapie oder in Kombination mit anderen Antipsychotika.

9. Verwendung nach einem der Ansprüche 1 bis 8, bei der das Arzneimittel 10 bis 300 mg 2-Cyano-10-(2-methyl-3-(methylamino)propyl)phenothiazin (I) oder pharmazeutisch annehmbare Salze davon enthält.

10. Verfahren zur Herstellung eines Arzneimittels, das zur Behandlung von Schlafstörungen verwendet werden kann, **dadurch gekennzeichnet, daß** man 2-Cyano-10-(2-methyl-3-(methylamino)propyl)phenothiazin (I) oder die pharmazeutisch annehmbaren Salze dieser Verbindung mit einem oder mehreren kompatiblen und pharmazeutisch annehmbaren Verdünnungsmitteln und/oder Hilfsstoffen vermischt.

## Revendications

1. Utilisation de 2-cyano-10-(2-méthyl-3-(méthylamino)propyl)phénothiazine (I) et ses sels pharmaceutiquement acceptables pour préparer un médicament destiné au traitement des troubles du sommeil.

2. Utilisation de 2-cyano-10-(2-méthyl-3-(méthylamino)propyl)phénothiazine (I) et ses sels pharmaceutiquement acceptables pour préparer un médicament destiné au traitement des troubles anxieux.

3. Utilisation de 2-cyano-10-(2-méthyl-3-(méthylamino)propyl)phénothiazine (I) et ses sels pharmaceutiquement acceptables pour préparer un médicament destiné au traitement des troubles de l'humeur.

4. Utilisation de 2-cyano-10-(2-méthyl-3-(méthylamino)propyl)phénothiazine (I) et ses sels pharmaceutiquement acceptables pour préparer un médicament destiné au traitement des troubles mixtes anxiété-dépression.

5. Utilisation de 2-cyano-10-(2-méthyl-3-(méthylamino)propyl)phénothiazine (I) et ses sels pharmaceutiquement acceptables pour préparer un médicament destiné au traitement d'un état psychotique aigu et chronique.

6. Utilisation de 2-cyano-10-(2-méthyl-3-(méthylamino)propyl)phénothiazine (I) et ses sels pharmaceutiquement acceptables pour préparer un médicament destiné au traitement de la dépendance et du sevrage à une substance.

7. Utilisation de 2-cyano-10-(2-méthyl-3-(méthylamino)propyl)phénothiazine (I) et ses sels pharmaceutiquement acceptables pour préparer un médicament destiné au traitement des évènements extrapyramidaux induits par les antipsychotiques.

8. Utilisation de 2-cyano-10-(2-méthyl-3-(méthylamino)propyl)phénothiazine (I) et ses sels pharmaceutiquement acceptables pour préparer un médicament destiné au traitement des dimensions symptomatiques aux cours des états psychotiques aigus ou chroniques en monothérapie ou en association avec d'autres antipsychotiques.

9. Utilisation selon l'une quelconque des revendications 1 à 8, dans laquelle le médicament comprend 10 à 300 mg de 2-cyano-10-(2-méthyl-3-(méthylamino)propyl)phénothiazine (I) ou ses sels pharmaceutiquement acceptables.

10. Procédé de préparation d'un médicament utile pour le traitement des troubles du sommeil, **caractérisé en ce que** l'on mélange la 2-cyano-10-(2-méthyl-3-(méthylamino)propyl)phénothiazine ou les sels pharmaceutiquement acceptables de ce composé avec un ou plusieurs diluants et/ou adjuvants compatibles et pharmaceutiquement acceptables.
